(19) Europäisches Patentamt

European Patent Office

Office européen des brevets

(11) **EP 0 696 454 B1**

(12) **EUROPÄISCHE PATENTSCHRIFT**

(45) Veröffentlichungstag und Bekanntmachung des Hinweises auf die Patenterteilung:
**29.09.1999 Patentblatt 1999/39**

(51) Int. Cl.$^6$: **A61K 31/565**, A61K 31/57
// (A61K31/57, 31:565)

(21) Anmeldenummer: **95250153.4**

(22) Anmeldetag: **28.06.1995**

(54) **Pharmazeutische Präparate zur Kontrazeption/Hormonsubstitution mit biogener Estrogenkomponente**

Pharmaceutical preparation for contraception/hormonsubstitution with biogenic estrogen components

Préparation pharmaceutique pour la contraception/traitement hormonal de substitution avec des composés oestrogéniques biogéniques

(84) Benannte Vertragsstaaten:
AT BE CH DE DK ES FR GB GR IE IT LI LU MC NL PT SE

(30) Priorität: **12.08.1994 DE 4429374**

(43) Veröffentlichungstag der Anmeldung:
**14.02.1996 Patentblatt 1996/07**

(73) Patentinhaber:
**Jenapharm GmbH & Co. KG**
**07745 Jena (DE)**

(72) Erfinder:
• **Oettel, Michael, Prof. Dr.**
  **D-07743 Jena (DE)**
• **Osterwald, Herman, Dr.**
  **D-37120 Bovenden (DE)**

• **Moore, Claudia, Dr.**
  **D-07747 Jena (DE)**
• **Gräser, Thomas, Dr.**
  **D-99097 Erfurt (DE)**

(74) Vertreter:
**Wablat, Wolfgang, Dr.Dr. et al**
**Patentanwalt,**
**Potsdamer Chaussee 48**
**14129 Berlin (DE)**

(56) Entgegenhaltungen:
WO-A-94/02103          WO-A-95/17895
DE-A- 4 313 926        DE-C- 4 308 406

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft Kontrazeptiva mit natürlicher Estrogenkomponente und hoher Zyklusstabilität, die gleichzeitig zur Hormonsubstitution in der Meno- und Postmenopause eingesetzt werden können und vorzugsweise in einem 28 Tage-Zyklus verabreicht werden.

Orale Kontrazeptiva stellen gegenwärtig und wahrscheinlich auch in Zukunft die häufigste Form der medikamentellen Geburtenkontrolle dar. Derartige Kontrazeptiva wurden erstmals in den frühen 60er Jahren auf dem Markt eingeführt und wurden seither durch intensive Forschungsarbeiten fortlaufend verbessert. Insbesondere gelang es, die notwendigen Dosierungen der Wirkstoffe zu reduzieren, so daß heute niedrig dosierte orale Kontrazeptiva zur Verfügung stehen. Abgesehen von den weniger bevorzugten Präparaten, die als Wirkstoff lediglich eine Gestagenkomponente enthalten ("Minipillen"), bestehen orale Kontrazeptiva in der Regel aus einer Estrogen- und einer Gestagenkomponente. Die Hormongaben erfolgen dabei in unterschiedlichsten Kombinationen und Dosierungen in Form von Stufen- oder Phasenpräparaten meist über einen Zeitraum von 21 Tagen, an den sich ein einnahmefreies Intervall von 6 Tagen anschließt.

Derartige Präparate zur Kontrazeption sind beispielsweise bekannt aus der DE-PS 32 29 612, der DE-PS 41 04 385, der US-PS 49 21 843 und der DE-PS 43 08 406.

Aus der WO-94/02103 A2 sind ovulationshemmende Mittel zur hormonalen Kontrazeption bekannt, bei denen in zwei Stufen zunächst eine Estrogenkomponente und danach eine Mischung aus einer Estrogenkomponente und einer Gestagenkomponente verabreicht werden. Als Estrogenkomponente werden biogene und/oder synthetische Estrogene verwendet.

Als Gestagenkomponente werden dabei von ihrer chemischen Struktur her sehr unterschiedliche Steroide verwendet. Typische Gestagene sind Steroide mit einer 17α-Ethinylgruppe und einer 13-Ethylgruppe, wie z.B. Desogestrel bzw. 3-Keto-Desogestrel, Gestoden, Levonorgestrel und Norgestimat, sowie die 13-Ethylverbindung Norethisteron bzw. Norethisteronacetat, ferner Gestagene ohne 17α-Ethinylgruppe und einer 13-Methyl-Gruppe wie z.B. Progesteron, Chlormadinonacetat, Cyproteronacetat, Medroxyprogesteronacetat, Megestrolacetat, Dydrogesteron, Dienogest usw. Die verschiedenen Gestagene sind hauptsächlich für die Ovulationshemmung und die ordnungsgemäße Transformation des Endometriums zuständig. Nach Absetzen dieser Geschlechtshormone kommt es somit zu der sogenannten (menstruationsähnlichen) Entzugsblutung.

Die Estrogenkomponente ist hauptsächlich für einen korrekten, zyklusgerechten Aufbau (Proliferation) des Endometriums (Gebärmutterschleimhaut) und damit für eine ausreichende Zykluskontrolle verantwortlich. Bevorzugt verwendet werden als Estrogenkomponenten das bereits seit über 40 Jahren bekannte synthetische Estrogen Ethinylestradiol oder dessen 3-Methylester, das Mestranol. Bei dem letzteren handelt es sich um ein Prodrug, das nach Resorption und Leberpassage in die Wirkform Ethinylestradiol umgewandelt wird. Andere synthetische Estrogene wie Stilbene oder Moxestrol haben sich infolge eines zu hohen toxischen Risikos nicht bewährt.

Durch die synthetische Einführung der 17α-Ethinylgruppe kann die Grundsubstanz 17β-Estradiol nicht in das viel weniger wirksame Estron umgewandelt werden und garantiert so seine hohe proliferative Wirkung an der Gebärmutterschleimhaut und die gute Zyklusstabilität, selbst bei extrem niedrigen Dosierungen von täglich 0.02 bis 0.03 mg. Allerdings weist Ethinylestradiol, bzw. das Prodrug Mestranol, eine Reihe schwerwiegender toxikologischer und pharmakologischer Nachteile auf. So wird Ethinylestradiol nur schlecht und individuell äußerst unterschiedlich im Magen-Darm-Trakt resorbiert, woraus eine ungenügende Bioverfügbarkeit resultiert. Weiterhin kommt es bei Verwendung von Ethinylestradiol zu einer signifikanten, dosisabhängigen Erhöhung des Thrombose-bzw. Thromboembolie-Risikos. Ferner wirkt Ethinylestradiol als suizidaler Hemmstoff auf bestimmte Isoenzyme des Cytochrom-P-450-Systems. Dies führt zu einer Inhibition eigener Abbau- und Metabolisierungswege. Da Gestagene und auch viele andere Wirkstoffe zum großen Teil über die gleichen Abbau- und Metabolisierungswege im Stoffwechsel laufen, führt dies nach wiederholter Applikation zu einer Kumulation der Wirkstoffe im Körper. Weitere unerwünschte Nebenwirkungen sind darüberhinaus z.B. die Blutdruckerhöhung infolge Induktion von Angiotensinogen bzw. Renin in der Leber, sowie das carcinogene Potential dieser Substanz (Bao Ting Zhu, Roy D, Liehr J: The carcinogenic activity of ethinyl estrogens is determined by both their hormonal characteristics and their conversion to catechol metabolites. Endocrinology 132: 577 - 583, 1993).

Trotz dieser erheblichen Nachteile konnten bisher keine alternativen Wirkstoffe für das Gebiet der oralen Kontrazeptiva gefunden werden. Es besteht daher nach wie vor ein dringender Bedarf, toxikologisch weniger bedenkliche Verbindungen für die Verwendung im Rahmen der Kontrazeption zur Verfügung zu stellen.

Eine toxikologisch weniger bedenkliche Verbindung, die sich anbieten würde, wäre das körpereigene 17β-Estradiol, das in mikronisierter Form oder in Form von Fettsäure-Estern (z.B. Estradiolvalerat) verabreicht werden kann. Dieses nicht-synthetische Hormon weist eine ganze Reihe von pharmakologisch-toxikologischen Vorteilen auf, wozu beispielsweise die blutdrucksenkende Wirkung (Clarkson TB: Experimental effects of estradiol versus progestins on arterial wall. Gynecol. Endocrinol. 6, Suppl. 1, 15; 1992), die Aufhebung der durch Endotheline bewirkten Gefäßkonstriktion (Jiang C, Poole-Wilson PA, Sarrel PM, Campbell S, Collins W:

Effect of 17β-estradiol on contraction, $Ca^{2+}$ current and intracellular free $Ca^{2+}$ in guinea-pig isolated cardiac myocytes. Br. J. Pharmacol. 104: 739, 1992; Jiang C, Sarrel PM, Poole-Wilson PA, Collins P: Acute effect of 17β-estradiol on rabbit coronary artery contractile responses to endothelin-1. Am. J. Physiol. 263: H271, 1992), die Hemmung der Lipidperoxidation in Zellmembranen und der LDL-Cholestrol-Oxidation als Initialschritte einer Atherosklerose (Mooradian AD: Antioxidant properties of steroids. J. Steroid Biochem. 45: 509-511, 1993), die Bremsung der Osteoclasten und damit des Knochenabbaus im Rahmen einer Osteoporose-Prophylaxe bzw. -Therapie, sowie die Linderung der senilen Demenz vom Alzheimer-Typ zu zählen sind.

Diese Vorteile des 17β-Estradiols haben zum häufigen Einsatz im Rahmen der Substitutionstherapie (Hormone Replacement Therapy, HRT) in der Meno- und Postmenopause bei Frauen geführt, wie z.B. in der DE-PS 32 13 248 und der DE-PS 26 45 307 beschrieben ist.

Bisher scheiterte der Einsatz dieses körpereigenen Hormons im Rahmen der Kontrazeption als alleiniger Estrogenbestandteil von Kombinationspräparaten in Verbindung mit einer Gestagenkomponente jedoch an dem Auftreten unakzeptabler Zyklusstörungen und konnte sich trotz vielfältiger Bemühungen nicht durchsetzen. Zwar gewährleistet in derartigen Kombinationspräparaten die Gestagenkomponente einen sicheren Kontrazeptionsschutz; da aber Gestagen durch Stimulierung lokaler Enzyme eine verstärkte Inaktivierung des Estradiols im Endometrium verursacht und dadurch die Estradiolwirkung auf das Endometrium stark reduziert ist, kommt es häufig zu Zwischenblutungen. Beschrieben sind lediglich Kombinationspräparate zur Kontrazeption, in denen neben synthetischen Estrogenen biogene Estrogene verwendet werden, wodurch sich der Vorteil ergibt, daß wesentlich niedrigere Wirkstoffkonzentrationen erforderlich sind (DE-PS 43 08 406). Ganz kann jedoch auch hier nicht auf den Einsatz synthetischer Estrogene verzichtet werden.

Da die Verwendung körpereigener Estrogene für die Kontrazeption bisher nicht in zufriedenstellendem Maße realisiert werden konnte, gelang es auch noch nicht, eine akzeptable "Pille" für Frauen in allen Lebensphasen, d.h. zur Kontrazeption im fertilen Lebensabschnitt und gleichzeitig zur Hormonsubstitution nach Erlöschen der Estradiolbiosynthese in den Ovarien, d.h. in der Meno- und Postmenopause zu entwickeln.

Ein weiterer Nachteil der bekannten zur Kontrazeption verwendeten Kombinations- und Sequenzpräparate ist, daß sich an die Einnahme von in der Regel 21 bis 27 Tagesdosiseinheiten eine entsprechend lange Einnahme-Pause anschließt, in der es zu einer die natürliche Monatsblutung simulierenden Entzugsblutung kommt (siehe beispielsweise DE-PS 32 29 612). So ist beispielsweise aus der US-PS 49 21 843 ein ovulationshemmendes Mittel bekannt, bei dem zwischen der Einnahme der letzten Hormon-Tagesdosiseinheiten der zweiten Hormonkomponente eine Einnahmepause von mindestens einem Tag, vorzugsweise zwei Tagen vorgesehen ist, die beispielsweise durch die Gabe von (einem) Placebo(s) überbrückt werden kann, ehe eine erneute Hormon-Tagesdosiseinheit, und zwar die erste der ersten Hormonkomponente des nächstfolgenden Zyklus eingenommen wird. Dies entspricht der in der Fachwelt vorherrschenden Meinung, wonach eine Einnahmepause von mindestens einem Tag oder aber eine signifikante Verringerung des wirksamen Estrogenspiegels als Voraussetzung für die Auslösung einer Entzugsblutung angesehen wird.

Das Absetzen des Estrogens, und sei es auch nur für einen Tag, kann jedoch zu Veränderungen der Durchblutung führen und somit Ursache für Kopfschmerzen (z.B. Migräneanfälle) sein. Ferner können kurzfristige Veränderungen anderer Stoffwechselparameter, beispielsweise der Hämostase, auftreten. Das in der DE-PS 41 04 385 beschriebene Kombinationspräparat sieht zwar die durchgehende Verabreichung einer Estrogenkomponente über den gesamten Zyklus vor, jedoch werden durch das dort vorgeschlagene Präparat nicht die mit der Verwendung biogener Estrogene verbundenen Schwierigkeiten überwunden. In diesem Fall bestehen also nach wie vor die Risiken, die mit der Verwendung endogener Estrogene in Kombinationspräparaten zur Kontrazeption verbunden sind.

Der Erfindung liegt die Aufgabe zugrunde, ein ovulationshemmendes Mittel der gattungsmäßigen Art dahingehend weiterzuentwickeln, daß bei hoher kontrazeptiver Sicherheit eine einwandfreie Zykluskontrolle unter zuverlässiger Vermeidung von Zwischenblutungen erreicht und Nebenwirkungen vermieden werden.

Eine weitere Aufgabe der vorliegenden Erfindung ist es, ein derartiges Mittel zur Verfügung zu stellen, das die Verwendung körpereigener Estrogene erlaubt.

Aufgabe der vorliegenden Erfindung ist es ferner, ein Mittel zur Verfügung zu stellen, daß sich sowohl zur Kontrazeption als auch zur Hormonsubstitution in der Meno- und Postmenopause bei Frauen verwenden läßt.

Diese Aufgaben werden erfindungsgemäß dadurch gelöst, daß ein dreistufiges Kombinationspräparat zur Kontrazeption/Hormonsubstitution mit insgesamt 28 Tagesdosiseinheiten zur Verfügung gestellt wird bestehend aus

einer ersten Stufe, die aus 3 oder 4 Tagesdosiseinheiten besteht, wobei jede Tagesdosiseinheit eine Zusammensetzung enthält, die als Wirkstoff mindestens eine biogene Estrogenkomponente enthält,

einer zweiten Stufe aus 20 bis 22 Tagesdosiseinheiten besteht, wobei jede Tagesdosiseinheit eine Zusammensetzung enthält, die als Wirkstoffe mindestens eine biogene Estrogen- und mindestens eine Gestagenkomponente aus der Gruppe der $C_{21}$-Gestagene einschließlich des Progesterons sowie des Dienogests, Desogestrels, 3-Keto-Desogestrels, Gestodens, Levonorgestrels, Norgestimats, Norethisterons, Norethisteronacetats und des Dydrogesterons aufweist, und

und einer dritten Stufe, die aus 3 oder 4 Tagesdosiseinheiten besteht, wobei jede Tagesdosiseinheit eine Zusammensetzung enthält, die als Wirkstoff mindestens eine biogene Estrogenkomponente aufweist, wobei

die zweite Stufe aus zwei Gruppen von Tagesdosiseinheiten besteht, wobei die erste Gruppe (a) 7 bis 9 Tagesdosiseinheiten umfaßt, und die zweite Gruppe (b) 12 bis 14 Tagesdosiseinheiten umfaßt, die Tagesdosiseinheiten beider Gruppen gleich Mengen mindestens einer Gestagenkomponente enthalten und die Tagesdosiseinheiten der Zweiten Gruppe eine größere Menge der biogenen Estrogenkomponente enthalten als die der ersten Gruppe.

Hierbei erfolgt eine zusätzliche Gabe an biogenen Estrogenen vorzugsweise an den Zyklustagen 12 bis 25. Die Menge an zusätzlich appliziertem Estradiolvalerat kann beispielsweise im Bereich von 1 bis 2 mg liegen. Bei der Verwendung anderer biogener Estrogene werden analoge Mengen appliziert, um den gewünschten Effekt zu erzielen.

Ferner sieht eine vorteilhafte Ausgestaltung der Erfindung vor, daß als biogene Estrogenkomponente 17β-Estradiol oder dessen Ester mit einer physiologisch verträglichen Säure oder ein 17β-Estradlolderivat, welches unter physiologischen Bedingungen 17β-Estradiol freisetzt oder eine Mischung aus den vorgenannten Verbindungen verwendet wird. Erfindungsgemäß wird das Estrogen über den gesamten Zyklus verabreicht. Dies gewährleistet eine bessere Kontrolle der ovariellen Follikel und die Vermeidung von eventuell auftretenden unerwünschten Nebenwirkungen bei einer kurzzeitigen Einnahmepause, wie bereits oben erörtert wurde.

Der Erfindung liegt die Erkenntnis zugrunde, daß es möglich ist, durch geeignete Wahl der Gestagenkomponente ein Kombinationspräparat zur Kontrazeption zu entwickeln, das die Verwendung biogener Estrogenkomponenten erlaubt bei einer gleichzeitig hohen kontrazeptiven Sicherheit, einer einwandfreien Zyklusstabilität und einer effektiven Vermeidung von Zwischenblutungen. Es war bekannt, daß die relativ unsichere Wirkung des körpereigenen Estrogens 17β-Estradiol oder seiner Ester auf das Endometrium (und die damit verbundenen nicht tolerierbaren Zyklusstörungen bei jüngeren Frauen) wie oben bereits erwähnt auf der metabolischen Umwandlung von Estradiol in das weniger wirksame Estron in den Endometrium-Zellen beruht. Das für die Umwandlung verantwortliche Enzym, die 17β-Dehydrogenase, wird von den bei Kombinationspräparaten gleichzeitig applizierten Gestagenen zusätzlich induziert. Es wurde nun gefunden, daß sich die verschiedenen Gestagene hinsichtlich dieses Effektes sehr stark unterscheiden. Dementsprechend ist es möglich, durch Identifizierung der Gestagene, die eine möglichst geringe Induktion der 17β-Dehydrogenase bewirken, Kombinationspräparate zu entwickeln, die als Estrogenkomponente ein biogenes Estrogen enthalten und zur sicheren Kontrazeption geeignet sind.

In einer bevorzugten Ausführungsform des erfindungsgemäßen Kombinationspräparates wird daher als Gestagenkomponente mindestens ein Gestagen eingesetzt, das eine möglichst geringe Induktion der 17β-Dehydrogenase bewirkt. Als besonders geeignet erwiesen sich die sogenannten C21-Gestagene sowie Progesteron. Besonders bevorzugt ist die Verwendung von Chlormadinonacetat oder Dienogest als Gestagenkomponente des erfindungsgemäßen Kombinationspräparates.

Es wurde weiterhin gefunden, daß Zyklusstörungen nach oraler Kontrazeption vorzugsweise in der zweiten Zyklushälfte auftreten und daß diese Zyklusstörungen durch zusätzliche tägliche Gabe von biogenen Estrogenen, beispielsweise Estradiolvalerat, in diesem Zeitraum vermieden werden können.

Das erfindungsgemäße Kombinationspräparat weist darüberhinaus den Vorteil auf, daß es aufgrund der Verwendung biogener Estrogene sowohl zur Kontrazeption als auch zur Hormonsubstitution in der Meno- und Postmenopause geeignet ist und daher eine akzeptale "Pille" für Frauen in allen Lebensphasen darstellt.

Ein wirksames erfindungsgemäßes Kombinationspräparat enthält beispielsweise folgende Wirkstoffe pro Tagesdosiseinheit:

| Stufe 1: | | 2.0 mg Estradiolvalerat |
|---|---|---|
| Stufe 2: | (a) | 2.0 mg Estradiol |
| | | 2.0 mg Dienogest |
| | (b) | 4.0 mg Estradiol |
| | | 2.0 mg Dienogest |
| Stufe 3: | | 2.o mg Estradiolvalerat |

Dabei erfolgt die Applikation der ersten Stufe beispielsweise über einen Zeitraum der ersten 4 Tage des Zyklus, die Applikation der ersten Gruppe (a) der zweiten Stufe über einen Zeitraum von 7 Tagen, die Applikation der zweiten Gruppe (b) der zweiten Stufe über einen Zeitraum von 14 Tagen und die Applikation der dritten Stufe über einen Zeitraum von 3 Tagen.

Die erfindungsgemäßen Wirkstoffkombinationen werden bevorzugt in Form von oral anwendbaren galenischen Zubereitungsformen appliziert. Als orale Zubereitungsformen kommen beispielsweise Tabletten, Dragees, Pillen oder Kapseln in Frage. Die Zubereitungsformen werden in an sich üblicher Weise unter Verwendung üblicher Hilfs- und Trägerstoffe hergestellt, wie sie beispielsweise im "Remington's Pharmaceutical Sciences Handbook, Hack Pub. Co., N.Y., USA" beschrieben sind.

Eine Variante der Applikation der erfindungsgemäßen Wirkstoffkombination stellt die Applikation in Form von Vaginalzäpfchen und Vaginalkapseln dar. Dazu werden Vaginalzäpfchen und Vaginalkapseln nach den üblichen Methoden unter Verwendung der üblichen Hilfsstoffe hergestellt.

Die erfindungsgemäßen Wirkstoffkombinationen können ferner in Form von transdermalen therapeutischen Systemen (TTS) appliziert werden. Dazu werden die erfindungsgemäßen Wirkstoffkombinationen in an sich bekannter Weise in ein TTS eingebracht. Das TTS kann beispielsweise auf Ionophorese oder Diffusion oder gegebenenfalls auf Kombination dieser Effekte beruhen.

Das TTS wird an geeigneter Stelle am Körper angebracht. Die Wirkstoffe werden dann transcutan appliziert, wobei die Applikationsrate durch die Größe der Fläche des TTS und durch die gegebenenfalls angelegte Spannung gesteuert wird.

Für die bevorzugte orale Applikation werden die erfindungsgemäßen Kombinationspräparate zur Kontrazeption und Hormonsubstitution zweckmäßig in Form einer pharmazeutischen Packung Zusammengefaßt, welche die tägliche Dosierung darstellende Formulierung in fortlaufender Reihenfolge unterbringt. Gegenstand der vorliegenden Erfindung sind somit auch pharmazeutische Packungen, die dadurch gekennzeichnet sind, daß sie Dosierungseinheiten in abgestimmter, festgelegter Reihenfolge enthalten, wobei die Reihenfolge den Stufen der täglichen Verabfolgung entspricht. Die pharmazeutische Packung kann beispielsweise in Form einer Tiefziehpackung hergestellt werden. Abwandlungen bezüglich der täglichen Dosierung, der Ausführungen der Applikationsformen, der Form der Packung etc. sind dem Fachmann geläufig.

In der vorliegenden Anmeldung werden folgende Definitionen verwendet:

"Menstruationszyklus" oder "Zyklus" bezieht sich auf die bekannte und wiederholt auftretende Menstruationssequenz bei der Frau im gebärfähigen Alter vor der Menopause von üblicherweise 28 Tagen Dauer.

"Tag eins" dieses Zyklus wird als der erste Tag der Menstruation definiert, und danach werden die Tage weitergezählt bis die Menstruation erneut eintritt; die Zahl der Tage beträgt normalerweise 28, kann in manchen Fällen jedoch etwas niedriger oder höher sein.

"Menopause" ist der Termin der letzten Menstruationsblutung im Leben der Frau, der retrospektiv nach 1-jährigem Ausbleiben der Regelblutung festgelegt wird. Es findet in den Ovarien kein Wachstum, kein Reifen und kein Platzen der Follikel mehr statt. Die körpereigene Estradiol-Produktion sinkt stark ab. "Postmenopause" bezeichnet den Lebensabschnitt nach der Menopause.

Der Begriff "Estrogen" umfaßt hier Hormone und alle Verbindungen, die die Aktivität eines Estrogens besitzen, d.h. die Fähigkeit besitzen, eine physiologische Reaktion hervorzurufen ähnlich der, die normalerweise von endogenen Estrogenen in weiblichen Erwachsenen ausgelöst wird, z.B. die Inhibierung der Sekretion von FSH.

"Estradiol" umfaßt gemäß vorliegender Definition $17\beta$-Estradiol und alle Ester des körpereigenen Estrogens.

Der Begriff "Gestagen" bezieht sich auf jede Verbindung, die eine dem Progesteron ähnliche progestagene Wirkung zeigt. Typische Gestagene sind die Steroide mit einer $17\alpha$-Ethinylgruppe und einer 13-Ethylgruppe wie z.B. Desogestrel bzw. 3-Keto-Desogestrel, Gestoden, Levonorgestrel und Norgestimat, sowie die 13-Ethylverbindung Norethisteron bzw. Norethisteronacetat, ferner Gestagene ohne $17\alpha$-Ethinylgruppe und einer 13-Methyl-Gruppe wie z. B. Progesteron, Chlormadinonacetat, Cyproteronacetat, Medroxyprogesteronacetat, Megestrolacetat, Dydrogesteron, Dienogest usw..

Die folgenden Beispiele veranschaulichen die vorliegende Erfindung:

Beispiel 1

(Zusammensetzung einer Tablette je Stufe)

1. Stufe (4 Tabletten)

| | |
|---|---|
| 2,0 mg | Estradiolvalerat- mikronisiert auf eine durchschnittliche Teilchengröße von 3 $\mu$m |
| 33,4 mg | Lactose |
| 17,2 mg | Maisstärke |
| 2,1 mg | Polyvinylpyrrolidon |
| 0,3 mg | Magnesiumstearat |
| 57,0 mg | Gesamtgewicht, das mit einer üblichen Zuckermischung auf etwa 75 mg ergänzt wird. |

2. Stufe: (a) (7 Tabletten)

| | |
|---|---|
| 2,0 mg | Estradiol- mikronisiert auf eine durchschnittliche Teilchengröße von 3 $\mu$m |
| 2,0 mg | Dienogest- mikronisiert |
| 33,4 mg | Lactose |

| 17,2 mg | Maisstärke |
| 2,1 mg | Polyvinylpyrrolidon |
| 0,3 mg | Magnesiumstearat |
| $\overline{57,0\ mg}$ | Gesamtgewicht, das mit einer üblichen Zuckermischung auf etwa 75 mg ergänzt wird. |

und anschließend (b) (14 Tabletten)

| 4,0 mg | Estradiol- mikronisiert auf eine durchschnittliche Teilchengröße von 3 µm |
| 2,0 mg | Dienogest- mikronisiert |
| 33,4 mg | Lactose |
| 17,2 mg | Maisstärke |
| 2,1 mg | Polyvinylpyrrolidon |
| 0,3 mg | Magnesiumstearat |
| $\overline{57,0\ mg}$ | Gesamtgewicht, das mit einer üblichen Zuckermischung auf etwa 75 mg ergänzt wird. |

3. Stufe: (3 Tabletten)

| 2,0 mg | Estradiolvalerat- mikronisiert auf eine durchschnittliche Teilchengröße von 3 µm |
| 33,4 mg | Lactose |
| 17,2 mg | Maisstärke |
| 2,1 mg | Polyvinylpyrrolidon |
| 0,3 mg | Magnesiumstearat |
| $\overline{57,0\ mg}$ | Gesamtgewicht, das mit einer üblichen Zuckermischung auf etwa 75 mg ergänzt wird. |

Beispiel 2

(Zusammensetzung einer Tablette je Stufe)

1. Stufe (4 Tabletten)

| 2,0 mg | Estradiolvalerat- mikronisiert auf eine durchschnittliche Teilchengröße von 3 µm |
| 33,4 mg | Lactose |
| 17,2 mg | Maisstärke |
| 2,1 mg | Polyvinylpyrrolidon |
| 0,3 mg | Magnesiumstearat |
| $\overline{57,0\ mg}$ | Gesamtgewicht, das mit einer üblichen Zuckermischung auf etwa 75 mg ergänzt wird. |

2. Stufe: (a) (7 Tabletten)

| 2,0 mg | Estradiol- mikronisiert auf eine durchschnittliche Teilchengröße von 3 µm |

| 2,0 mg | Dienogest- mikronisiert |
| 33,4 mg | Lactose |
| 17,2 mg | Maisstärke |
| 2,1 mg | Polyvinylpyrrolidon |
| 0,3 mg | Magnesiumstearat |
| $\overline{57,0\ mg}$ | Gesamtgewicht, das mit einer üblichen Zuckermischung auf etwa 75 mg ergänzt wird. |

und anschließend (b) (14 Tabletten)

| 3,0 mg | Estradiol- mikronisiert auf eine durchschnittliche Teilchengröße von 3 µm |
| 2,0 mg | Dienogest- mikronisiert |
| 33,4 mg | Lactose |
| 17,2 mg | Maisstärke |
| 2,1 mg | Polyvinylpyrrolidon |
| 0,3 mg | Magnesiumstearat |
| $\overline{57,0\ mg}$ | Gesamtgewicht, das mit einer üblichen Zuckermischung auf etwa 75 mg ergänzt wird. |

3. Stufe: (3 Tabletten)

| 2,0 mg | Estradiolvalerat- mikronisiert auf eine durchschnittliche Teilchengröße von 3 µm |
| 33,4 mg | Lactose |
| 17,2 mg | Maisstärke |
| 2,1 mg | Polyvinylpyrrolidon |
| 0,3 mg | Magnesiumstearat |
| $\overline{57,0\ mg}$ | Gesamtgewicht, das mit einer üblichen Zuckermischung auf etwa 75 mg ergänzt wird. |

Beispiel 3

(Zusammensetzung einer Tablette je Stufe)

1. Stufe (4 Tabletten)

| 1,5 mg | Estradiolvalerat- mikronisiert auf eine durchschnittliche Teilchengröße von 3 µm |
| 33,4 mg | Lactose |
| 17,2 mg | Maisstärke |
| 2,1 mg | Polyvinylpyrrolidon |
| 0,3 mg | Magnesiumstearat |
| $\overline{57,0\ mg}$ | Gesamtgewicht, das mit einer üblichen Zuckermischung auf etwa 75 mg ergänzt wird. |

2. Stufe: (a) (7 Tabletten)

| | |
|---|---|
| 1,5 mg | Estradiol- mikronisiert auf eine durchschnittliche Teilchengröße von 3 μm |
| 2,0 mg | Dienogest- mikronisiert |
| 33,4 mg | Lactose |
| 17,2 mg | Maisstärke |
| 2,1 mg | Polyvinylpyrrolidon |
| 0,3 mg | Magnesiumstearat |
| 57,0 mg | Gesamtgewicht, das mit einer üblichen Zuckermischung auf etwa 75 mg ergänzt wird. |

und anschließend (b) (14 Tabletten)

| | |
|---|---|
| 2,5 mg | Estradiol- mikronisiert auf eine durchschnittliche Teilchengröße von 3 μm |
| 2,0 mg | Dienogest- mikronisiert |
| 33,4 mg | Lactose |
| 17,2 mg | Maisstärke |
| 2,1 mg | Polyvinylpyrrolidon |
| 0,3 mg | Magnesiumstearat |
| 57,0 mg | Gesamtgewicht, das mit einer üblichen Zuckermischung auf etwa 75 mg ergänzt wird. |

3. Stufe: (3 Tabletten)

| | |
|---|---|
| 1,5 mg | Estradiolvalerat- mikronisiert auf eine durchschnittliche Teilchengröße von 3 μm |
| 33,4 mg | Lactose |
| 17,2 mg | Maisstärke |
| 2,1 mg | Polyvinylpyrrolidon |
| 0,3 mg | Magnesiumstearat |
| 57,0 mg | Gesamtgewicht, das mit einer üblichen Zuckermischung auf etwa 75 mg ergänzt wird. |

Beispiel 4

Die Zusammensetzung der Tabletten ist wie bei den Beispielen 1 bis 3, mit dem Unterschied, daß anstelle von Dienogest das Chlormadinonacetat in gleicher Dosierung verwendet wird.

Beispiel 4 a

Die Zusammensetzung der Tabletten ist wie bei den Beispielen 1 bis 3, mit dem Unterschied, daß anstelle von Dienogest das Desogestrel in einer Dosierung von 0,150 mg verwendet wird.

Beispiel 4 b

Die Zusammensetzung der Tabletten ist wie bei den Beispielen 1 bis 3, mit dem Unterschied, daß anstelle von Dienogest das Levonorgestrel in einer Dosierung von 0,100 mg verwendet wird.

Beispiel 4 c

Die Zusammensetzung der Tabletten ist wie bei den Beispielen 1 bis 3, mit dem Unterschied, daß anstelle von Dienogest das Gestoden mit einer Dosierung von 0,075 mg verwendet wird.

Beispiel 4 d

Die Zusammensetzung der Tabletten ist wie bei den Beispielen 1 bis 3, mit dem Unterschied, daß anstelle von Dienogest das Norgestimat mit einer Dosierung von 0,100 mg verwendet wird.

Beispiel 5

Zusammensetzung der Tabletten wie in den Beispielen 1 bis 3, mit dem Unterschied, daß anstatt Dienogest Progesteron verwendet wird, wobei die Dosierung in der zweiten Stufe erst 200 mg beträgt und dann auf 400 mg gesteigert wird.

Beispiel 6

Zusammensetzung der Tabletten wie in den Beispielen 1 bis 3, mit dem Unterschied, daß anstatt Dienogest Progesteron verwendet wird, wobei die Dosierung in der zweiten Stufe erst 300 mg beträgt und dann auf 400 mg gesteigert wird.

Beispiel 7

Die Zusammensetzungen der Tabletten erfolgt wie in den Beispielen 1 bis 6 dargestellt, wobei

die erste Stufe 3 Tabletten umfaßt,

| die zweite Stufe für und für | (a) 7 Tabletten (b) 14 Tabletten und |
|---|---|

die dritte Stufe 4 Tabletten umfaßt.

Beispiel 8

Die Zusammensetzungen der Tabletten erfolgt wie in den Beispielen 1 bis 6 dargestellt, wobei

die erste Stufe 4 Tabletten umfaßt,

| die zweite Stufe für und für | (a) 7 Tabletten (b) 14 Tabletten und |
|---|---|

die dritte Stufe 3 Tabletten umfaßt.

Beispiel 9

Die Zusammensetzungen der Tabletten erfolgt wie in den Beispielen 1 bis 6 dargestellt, wobei

die erste Stufe 4 Tabletten umfaßt,

die zweite Stufe für   (a) 9 Tabletten
und für            (b) 12 Tabletten und

die dritte Stufe 3 Tabletten umfaßt.

Beispiel 10

Die Zusammensetzungen der Tabletten erfolgt wie in den Beispielen 1 bis 6 dargestellt, wobei

die erste Stufe 3 Tabletten umfaßt,

die zweite Stufe für   (a) 9 Tabletten
und für            (b) 12 Tabletten und

die dritte Stufe 4 Tabletten umfaßt.

Beispiel 11

Die Zusammensetzungen der Tabletten erfolgt wie in den Beispielen 1 bis 6 dargestellt, wobei

die erste Stufe 4 Tabletten umfaßt,

die zweite Stufe für   (a) 8 Tabletten
und für            (b) 13 Tabletten und

die dritte Stufe 3 Tabletten umfaßt.

Beispiel 12

Die Zusammensetzungen der Tabletten erfolgt wie in den Beispielen 1 bis 6 dargestellt, wobei

die erste Stufe 3 Tabletten umfaßt,

die zweite Stufe für   (a) 8 Tabletten
und für            (b) 13 Tabletten und

die dritte Stufe 4 Tabletten umfaßt.

## Patentansprüche

1. Kombinationspräparat zur Kontrazeption/Hormonsubstitution, bestehend aus drei Stufen mit insgesamt 28 Tagesdosiseinheiten, dadurch gekennzeichnet, daß

die erste Stufe aus 3 oder 4 Tagesdosiseinheiten besteht, wobei jede Tagesdosiseinheit eine Zusammensetzung enthält, die als Wirkstoff mindestens eine biogene Estrogenkomponente aufweist,

die zweite Stufe aus 20 bis 22 Tagesdosiseinheiten besteht, wobei jede Tagesdosiseinheit eine Zusammensetzung enthält, die als Wirkstoffe mindestens eine biogene Estrogen- und mindestens eine Gestagenkomponente aus der Gruppe der $C_{21}$-Gestagene einschließlich des Progesterons sowie des Dienogests, Desogestrels, 3-Keto-Desogestrels, Gestodens, Levonorgestrels, Norgestimats, Norethisterons, Norethisteronacetats und des Dydrogesterons aufweist, und

die dritte Stufe aus 3 oder 4 Tagesdosiseinheiten besteht, wobei jede Tagesdosiseinheit eine Zusammensetzung enthält, die als Wirkstoff mindestens eine biogene Estrogenkomponente enthält, wobei

die zweite Stufe aus zwei Gruppen von Tagesdosiseinheiten besteht, wobei die erste Gruppe (a) 7 bis 9 Tagesdosiseinheiten umfaßt, und die zweite Gruppe (b) 12 bis 14 Tagesdosiseinheiten umfaßt, die Tagesdosiseinheiten beider Gruppen gleiche Mengen mindestens einer Gestagenkomponente enthalten und die Tagesdosiseinheiten der zweiten Gruppe eine größere Menge der biogenen Estrogenkomponente enthalten als die der ersten Gruppe.

2. Kombinationspräparat zur Kontrazeption/Hormonsubstitution gemäß Anspruch 1, dadurch gekennzeichnet, daß

die biogene Estrogenkomponente 17β-Estradiol oder dessen Ester mit einer physiologisch verträglichen Säure oder ein 17β-Estradiolderivat, welches unter physiologischen Bedingungen 17β-Estradiol freisetzt oder eine Mischung aus den vorgenannten Verbindungen ist.

3. Kombinationspräparat zur Kontrazeption/Hormonsubstitution gemäß Anspruch 1, dadurch gekennzeichnet, daß die Gestagenkomponente ein C21-Gestagen oder Progesteron oder eine Mischung der beiden Verbindungen enthält.

4. Kombinationspräparat zur Kontrazeption/Hormonsubstitution gemäß Anspruch 1 dadurch gekennzeichnet, daß die Geotagenkomponente Chlormadinonacetat oder Dienogest oder eine Mischung der beiden Verbindung enthält.

## Claims

1. A compound preparation for contraception/hormone replacement, consisting of three steps and a total of 28 daily doses, characterized in that

step 1 consists of 3 or 4 daily doses, each containing a preparation in which the active ingredient is at least one biogenous estrogen component,

step 2 consists of 20 to 22 daily doses, each containing a preparation in which the active ingredients are at least one biogenous estrogen and one gestagen component from the group of $C_{21}$-gestagens including progesterone, as well as dienogest, desogestrel, 3-keto desogestrel, gestodene, levonorgestrel, norgestimate, norethisterone, norethisterone acetate and dydrogesterone, and

step 3 consists of 3 or 4 daily doses, each containing a preparation in which the active ingredient is at least one biogenous estrogen component, and where

the second step consists of two groups of daily doses, the first group (a) comprising 7 to 9 daily doses and the second group (b) comprising 12 to 14 daily doses, the daily doses in both groups containing equal quantities of at least one gestagen component and the daily doses of the second group containing a greater quantity of the biogenous estrogen component than the first group.

2. The compound preparation for contraception/hormone replacement according to Claim 1 wherein

the biogenous estrogen component is 17b-estradiol or its ester with a physiologically acceptable acid, or a 17b-estradiol derivative that releases 17b-estradiol in physiological conditions, or a mixture of said compounds.

3. The compound preparation for contraception/hormone replacement according to Claim 1 wherein the gestagen component is a $C_{21}$-gestagen or progesterone, or a mixture of the two compounds.

4. The compound preparation for contraception/hormone replacement according to Claim 1 wherein the gestagen component contains chlormadinon acetate or dienogest, or a mixture of the two compounds.

**Revendications**

1. Préparation composite pour la contraception/substitution hormonale comprenant 3 étages avec au total 28 unités de dosage journalières caractérisée en ce que :

. le premier étage comporte 3 ou 4 unités de dosage journalières, chaque unité de dosage journalière comprenant une composition présentant en tant que substance active au moins un oestrogène biogène.

. le deuxième étage comprend de 20 à 22 unités de dosage journalières, chaque unité de dosage journalière comprenant une composition, laquelle comprend en tant que principe actif au moins un oestrogène biogène et au moins un gestagène choisi dans le groupe des composés gestagènes en C 21 incluant la progestérone ainsi que le dienogeste, le désogestrel le cétodésogestrel, le gestodène, le lévonorgestrel, le norgestimate, la noréthistérone, l'acétate de noréthistérone et la dydrogestérone.

. le troisième étage comprend 3 ou 4 unités de dosage journalières, chaque unité de dosage journalière comprenant une composition, laquelle comprend en tant que principe actif au moins un oestrogène biogène,

le deuxième étage comprenant deux groupes d'unités de dosage journalières, dont le premier groupe (a) comprend 7 à 9 unités de dosage journalières et le deuxième groupe (b) comprend 12 à 14 unités de dosages journalières, les unités de dosage journalières des deux groupes comprenant en quantité égale au moins un composé gestagène et les unités de dosage journalières du deuxième groupe comprenant un quantité plus importante de l'oestrogène biogène que le premier groupe.

2. Préparation composite pour la contraception/substitution hormonale selon la revendication caractérisée en ce que l'oestrogène biogène est le 17β-oestradiol ou son ester avec un acide physiologiquement acceptable ou un dérivé de 17β-oestradiol qui libère en conditions physiologiques le 17β oestradiol, ou un mélange de ces composés.

3. Préparation composite pour la contraception/substitution hormonale selon la revendication 1 caractérisée en ce que le composé gestagène contient un gestagène en $C_{21}$ de la progestérone ou un mélange des deux composés.

4. Préparation composite pour la contraception/substitution hormonale selon la revendication 1, caractérisée en ce que le composé gestagène contient de l'acétate de chlormadinone ou du diénogeste ou un mélange de ces deux composés.